(19) Europäisches Patentamt
European Patent Office
Office européen des brevets

(11) Veröffentlichungsnummer: **0 166 901**
**B1**

(12) # EUROPÄISCHE PATENTSCHRIFT

(45) Veröffentlichungstag der Patentschrift: **19.07.89**

(51) Int. Cl.$^4$: **C 07 C103/183**

(21) Anmeldenummer: **85105309.0**

(22) Anmeldetag: **02.05.85**

(54) **Verfahren zur Herstellung von D,L-Carnitinamidchlorid.**

(30) Priorität: **01.06.84 DE 3420391**

(43) Veröffentlichungstag der Anmeldung: **08.01.86 Patentblatt 86/02**

(45) Bekanntmachung des Hinweises auf die Patenterteilung: **19.07.89 Patentblatt 89/29**

(84) Benannte Vertragsstaaten: **AT BE CH FR GB IT LI NL SE**

(56) Entgegenhaltungen:
**US-A- 4 070 394**
**CHEMICAL ABSTRACTS, Band 59, Nr. 10, 11. November 1963, Spalte 11660-g, Columbus, Ohio, US; & JP - A - 63 23 (TAKEDA CHEMICAL INDUSTRIES, LTD.) 09-01-1963**

(73) Patentinhaber: **Degussa Aktiengesellschaft**
**Weissfrauenstrasse 9**
**D-6000 Frankfurt am Main 1 (DE)**

(72) Erfinder: **Klenk, Herbert, Dr. Dipl.-Chem.**
**Gleiwitzerstrasse 21**
**D-6450 Hanau 9 (DE)**

EP 0 166 901 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Die Erfindung betrifft ein neues Verfahren zur Herstellung von D, L-Carnitinamidchlorid in hoher Reinheit und guter Ausbeute.

D, L-Carnitinamidchlorid entspricht der Strukturformel (I).

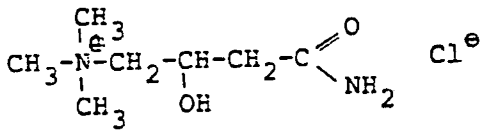

(I)

Es ist das D, L-γ-trimethylammonium-β-hydroxybutyramidchlorid.

D, L-Carnitinamidchlorid ist ein wichtiges Zwischenprodukt für die Herstellung von L-Carnitinamid und damit auch zur Herstellung von L-Carnitin (BE-PS 877 609). Beide Verbindungen, insbesondere aber das L-Carnitin, besitzen eine große therapeutische Bedeutung (Römpps Lexikon der Chemie, 8. Aufl., S. 607).

Es ist bekannt, das D,L-Carnitinamidchlorid durch Hydrolyse von isoliertem Carnitinnitril in konzentrierter Salzsäure und anschließender Fällung aus großen Mengen Isopropanol und Aceton herzustellen. Die Reaktionszeiten betragen dabei mehr als 46 Stunden. Außerdem besteht durch die Gegenwart einer konzentrierten Säure die Gefahr einer Weiterreaktion zu dem an dieser Stelle unerwünschten Carnitin. Aus diesen Gründen hat dieses Verfahren keinen Eingang in die Technik gefunden (BE-PS 659 194).

Es ist weiter bekannt, das Amid durch Umsetzung des Ethylesters von Carnitinchlorid mit Ammoniaklösung in einer 40-stündigen Reaktion herzustellen. Auch dieses Verfahren hat keinen Eingang in die Technik gefunden (Takeda Kenkyusho Nempo 22, 13-18 (1963)).

Außerdem ist es bekannt, bei der Herstellung des D, L-Carnitinamidchlorids von isoliertem Carnitinnitril auszugehen und beispielsweise mit doppelt äquivalenten Mengen Ammoniak und Wasserstoffperoxid in einer 20-stündigen Reaktion umzusetzen. Auch dieses Verfahren hat keinen Eingang in die Technik gefunden, da die dabei zu verwendenden großen Mengen an Base zu Schwierigkeiten bei der Isolierung des Carnitinamids führten, wenn eine Salzbildung durch Neutralisation mit Säure notwendig wurde. Außerdem entstanden Umweltprobleme bei der Eindampfung der Lösung. Weiter standen die langen Reaktionszeiten und die Notwendigkeit, ein isoliertes Nitril einzusetzen, der Überführung in den technischen Maßstab im Wege (JP-PS 38-23 (63)).

Schließlich ist noch die Umsetzung von 2,3-Epoxypropyltrimethylammoniumchlorid mit einer Cyanoverbindung zum entsprechenden Nitril bekannt, wobei aber zur Erzielung guter Ergebnisse von einer Epoxidverbindung ausgegangen werden muß, die nach McClure in einem organischen Medium hergestellt worden ist (US-Pat. 4 070 394) McClure, J. Org. Chem. 35, 2 059 (1970). Die Herstellung des Carnitinamidchlorids wird in dieser Patentschrift nicht beschrieben.

Es bestand daher ein dringendes Bedürfnis nach einem technisch gut durchführbaren und wirtschaftlich interessanten Verfahren zur Herstellung von D,L-Carnitinamidchlorid.

Es wurde nun gefunden, daß man D,L-Carnitinamidchlorid in vorteilhafter Weise herstellen kann, wenn man in einer ersten Reaktionsstufe 2,3-Epoxypropyltrimethylammoniumchlorid in wäßriger Lösung mit 0,8 bis 2,0 Blausäure bei einem pH-Wert oberhalb 7 und bei einer Temperatur zwischen 20 und 100 °C umsetzt und das entstandene Nitril ohne Zwischenisolierung in der Reaktionslösung mit 1,5 bis 5 Äquivalenten wäßrigem Wasserstoffperoxid bei einer Temperatur zwischen 30 und 100 °C behandelt und das Reaktionsprodukt in ansich bekannter Weise isoliert.

Eine wesentliche Bedingung bei der Durchführung des Verfahrens ist, daß die Reaktion in wäßriger Lösung erfolgt. Ausgangsprodukt der Synthese ist das 2,3-Epoxypropyltrimethylammoniumchlorid, welches aber nicht notwendigerweise nach McClure hergestellt sein muß. Vorzugsweise geht man sogar von einer Epoxyverbindung aus, die nach dem Verfahren gemäß DE-OS 31 03 713 hergestellt worden ist. Die Umsetzung erfolgt erfindungsgemäß mit Blausäure. Dabei kann reine Blausäure oder eine wäßrige Blausäure mit Gehalten von 1 bis 100 % Blausäure verwendet werden. Die Umsetzung läuft bei einem pH-Wert oberhalb 7 ab. Geht man von einer Epoxyverbindung aus, die gemäß der DE-OS 31 03 713 hergestellt worden ist, so stellt sich ein pH-Wert oberhalb von 8, im allgemeinen zwischen 9,5 bis 12, ein. Im Bedarfsfalle kann der pH-Wert durch Zugabe einer geringfügigen Menge einer Base eingestellt werden.

Die Temperatur, bei der die Reaktion durchgeführt wird, ist nicht kritisch. Sie sollte nur so gewählt werden, daß gute Umsätze erreichbar sind. Üblicherweise reicht dazu eine Reaktionstemperatur oberhalb von 20 °C aus. Der bevorzugte Temperaturbereich liegt etwa zwischen + 20 und 100 °C.

Obgleich das Molverhältnis zwischen Epoxyverbindung und Blausäure frei gewählt werden kann, wird üblicherweise ein Verhältnis von Epoxyverbindung : Blausäure wie 1 : 0,8 bis 2, bevorzugt von 1 : 0,95 bis 1,25, eingehalten.

Ein weiteres wesentliches Merkmal der Erfindung ist, daß das in wäßriger Lösung entstandene Nitrilchlorid nicht isoliert, sondern direkt weiterverarbeitet wird. Dazu wird Wasserstoffperoxid zugegeben. Üblicherweise wird eine wäßrige Lösung von Wasserstoffperoxid zwischen 3 bis 90 Gew.-% verwendet, bevorzugt zwischen 20 bis 90 Gew.-%.

Bei dem erfindungsgemäßen Verfahren werden üblicherweise, bezogen auf das eingesetzte Nitril, überschüssige Mengen Wasserstoffperoxid eingesetzt. Beispielsweise werden 1,5 bis 5 Äquivalente Wasserstoffperoxid bezogen auf Nitril verwendet. Bevorzugt setzt man 2 bis 3,5 Äquivalente ein.

Obwohl die Temperatur für die zweite Stufe der Reaktion nicht kritisch ist, sollte sie so gewählt werden, daß gute Umsätze erreicht werden, aber doch noch keine Weiterreaktion zu anderen Folgeprodukten, wie beispielsweise Carnitin, erfolgt. So werden üblicherweise Temperaturen zwischen 30 und 100 °C gewählt, bevorzugt zwischen 40 und 80 °C. Das Ende der Umsetzung ist beispielsweise leicht daran zu erkennen, daß eine bei der Reaktion vorhandene Gasbildung beendet ist.

Die Isolierung des so in Lösung hergestellten D, L-Carnitinamidchlorids geschieht in an sich bekannter Weise. Wegen der in Wasser sehr hohen Löslichkeit des D, L-Carnitinamidchlorids wird üblicherweise zunächst weitgehend Wasser abgedampft und dann das D,L-Carnitinamidchlorid durch Zugabe eines organischen Lösungsmittels ausgefällt. Beispielsweise können hierfür Methanol, Ethanol, Propanole, Butanole oder andere Alkohole oder Aceton oder andere Ketone verwendet werden. Es kann aber auch z. B. die gesamte wäßrige Lösung durch Sprühkristallisation in D, L-Carnitinamidchlorid überführt werden.

## Beispiel

Zu 253 g einer 59,8 %igen (1,0 Mol) Lösung von 2,3-Epoxypropyltrimethyl-ammoniumchlorid, das nach dem Verfahren gemäß DE-OS 31 03 713 hergestellt worden war und einen pH-Wert von 11 aufzeigt, werden in einer Rührapparatur bei einer Temperatur von 50 °C 27,8 g (1,03 Mol) wasserfreier Blausäure während einer Stunde zudosiert. Nach einer weiteren Stunde Nachreaktion werden ohne weitere Maßnahme bei derselben Temperatur in die entstandene Lösung 111,7 g 70 %iges Wasserstoffperoxid (2,3 Mol) während etwa 3 Stunden eindosiert und weitere 3 Stunden bei 50 °C nachreagiert. Danach werden bei etwa 70 mbar 110 g Wasser abdestilliert und der entstandene Sirup mit 300 ml Ethanol versetzt und ausgerührt. Nach Abkühlung auf 0 °C wird die entstandene Suspension abgesaugt und der erhaltene Feststoff getrocknet. Nach dem Trocknen erhält man 178,5 g (91 % bezogen auf eingesetztes Epoxid) eines blütenweißen Feststoffes vom Schmelzpunkt 210 °C.

Die Elementaranalyse zeigt folgende Ergebnisse :

| | | | | |
|---|---|---|---|---|
| ber. | C 42,7 | H 8,7 | N 14,2 | D 18,0 % |
| gef. | C 42,65 | H 8,8 | N 14,15 | D 17,9 % |

## Patentanspruch

Verfahren zur Herstellung von D, L-Carnitinamidchlorid (I),

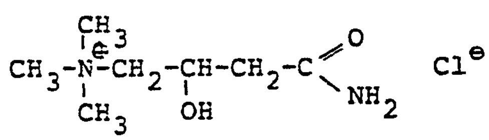

(I)

dadurch gekennzeichnet, daß man in einer ersten Reaktionsstufe 2,3-Epoxypropyltrimethyl-ammoniumchlorid in wäßriger Lösung mit 0,8 bis 2,0 Äquivalenten Blausäure bei einem pH-Wert oberhalb 7 und nicht über 11 sowie bei einer Temperatur zwischen 20 und 100 °C umsetzt und das entstandene Nitril ohne Zwischenisolierung und ohne Zugabe von Base in der Reaktionslösung mit 1,5 bis 5 Äquivalenten wäßrigem Wasserstoffperoxid bei einer Temperatur zwischen 30 und 100 °C behandelt und das Reaktionsprodukt in an sich bekannter Weise isoliert.

## Claim

Process for the preparation of D,L-carnitinamide chloride (I)

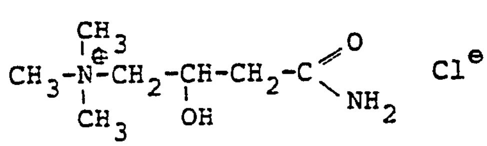

(I)

characterised in that, in a first reaction stage, 2,3-epoxypropyltrimethylammonium chloride is reacted in

aqueous solution with 0.8 to 2.0 equivalents of hydrocyanic acid at a pH above 7 and not above 11 and at a temperature between 20 and 100 °C, and the resulting nitrile is, without intermediate isolation and without addition of base, treated in the reaction solution with 1.5 to 5 equivalents of aqueous hydrogen peroxide at a temperature between 30 and 100 °C, and the reaction product is isolated in a manner known per se.

**Revendication**

Procédé d'obtention du chlorure de D. L carnitinamide I

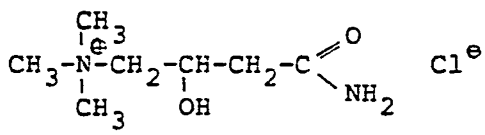

(I)

caractérisé en ce que l'on condense dans une première étape du procédé le chlorure de 2,3-époxypropyl triméthylammonium en solution aqueuse avec 0,8 à 2,0 équivalents d'acide cyanhydrique à une valeur de pH au-dessus de 7 et qui n'est pas supérieure à 11, ainsi qu'à une température comprise entre 20 et 100 °C, et que l'on traite le nitrile résultant sans isolement intermédiaire et sans addition d'une base dans la solution réactionnelle avec 1,5 à 5 équivalents de peroxyde d'hydrogène aqueux à une température comprise entre 30 et 100 °C et que l'on isole le produit de la réaction d'une manière connue en soi.